# EUROPEAN PATENT APPLICATION

(11) **EP 2 397 123 A1**
(43) Date of publication of application: **21.12.2011**
(21) Application number: 11167103.8
(22) Date of filing: 06.07.2007
(51) Int. Cl.: A61K 9/51, A61K 47/36, A61K 48/00, C12N 15/11

(54) **Nanoparticles for nucleic acid delivery**

(30) Priority: 07.07.2006 US 819209 P
(62) Divisional of application: 10187562.3
(71) Applicant: Aarhus Universitet, 8000 Aarhus C (DK)
(72) Inventor: Howard, Kenneth Alan, 8200, Aarhus N (DK); Kjems, Jørgen, 8240, Risskov (DK); Liu, Xiudong, 116023, Dalian (CN); Besenbacher, Flemming, 8210, Aarhus V (DK)
(74) Representative: Plougmann & Vingtoft A/S

(57) **Abstract**

The present invention provides chitosan/RNA nanoparticles that are useful as research tools or medicaments. Preferably, the RNA part of the nanoparticle is a siRNA capable of modulating the expression of a target mRNA. The invention also provides methods for the preparation of chitosan/RNA nanoparticles.

## Description

### Background

Delivery of nucleic acids into target cells is of interest for various reasons. For example, the use of expression plasmids and antisense molecules as gene therapy drugs has been hampered by poor delivery techniques. Furthermore, an improved delivery technique could expand the use of so-called aptamers, to also include cellular targets. Aptamers are nucleic acids that fold into a three-dimensional structure that allow them to interact with target molecules at high affinity and specificity.

Another class of nucleic acids that has attracted massive attention recently is small interfering RNAs and micro RNAs. These double stranded RNA complexes can mediate various modifications of target nucleic acids in the cell. In this process, the antisense strand of the complex acts as a guide, as the antisense strand can hybridise to target nucleic acids that have stretches of sequence complementarily to the antisense strand.

RNA-mediated knockdown of protein expression at the messenger RNA (mRNA) level offers a new therapeutic strategy to overcome disease. The process by which small interfering RNA (siRNA) modulate enzymatic-induced cleavage of homologous mRNA and concomitant interruption of gene expression (RNA interference) has been extensively studied as a tool for investigating cellular processes in mammalian cells. RNA interference has been demonstrated using siRNA directed against genes responsible for viral pathogenesis, cancer and inflammatory conditions. As a consequence, the possibility to silence genes implicated in disease using siRNA has led to a rapidly evolving area in drug discovery.

RNA molecules may also induce transcriptional silencing. RNA-induced transcriptional silencing is a form of RNA interference by which short RNA molecules - microRNA (miRNA) or small interfering RNA (siRNA) - trigger the downregulation of transcription of a particular gene or genomic region. This is usually accomplished by modification of histones, often by methylation, or by the induction of heterochromatin formation.

The effectiveness of a drug is determined by the ability to migrate through the body and reach target sites at therapeutically relevant levels. Injection of naked siRNA into mice has resulted in localized gene knockdown. A drawback, however, is the requirement for high doses due to RNA instability and non-specific cellular uptake and impracticality of this method for human use. Viral and non-viral delivery systems have been developed in order to overcome extracellular and intracellular barriers that restrict therapeutic use of nucleic acid-based drugs.

Polyelectrolyte nanoparticles formed by self assembly of plasmid DNA with polycations, widely used in gene delivery, have been adopted for siRNA use. Cationic polymer and lipid-based siRNA vectors have been shown to enter cells and mediate specific RNA interference *in vitro* and *in vivo*. The effectiveness of nanoparticle systems is limited by rapid clearance from the circulation either by mononuclear phagocytic cell capture or non-specific cationic interactions with biological membranes and connective tissue. An alternative approach for both local and systemic drug delivery is exploitation of mucosal routes, e.g. nasal, to avoid the first pass hepatic clearance mechanism associated with intravenous administration. The polysaccharide chitosan, successfully used for nasal drug delivery due to mucoadhesive and mucosa permeation properties, has been utilised in the formation of polyelectrolyte nanoparticles containing plasmids for gene expression in respiratory sites and systemic tissue.

### Summary of the invention

The present invention relates to the field of nucleic acids and their delivery into cells. In particular the invention relates to nanoparticles comprising RNA molecules and methods for the preparation of such nanoparticles using chitosan. These are of interest, because they can be used in basic research and potentially as medicaments.

### Detailed description of the invention

The term chitosan as used herein has the same meaning as generally in the art. I.e. chitosan refers to a linear polysaccharide composed of randomly distributed ß-(1-4)-linked D-glucosamine (deacetylated unit) and N-acetyl-D-glucosamine (acetylated unit). Chitosan is typically produced by deacetylation of chitin, which is the structural element in the exoskeleton of crustaceans (crabs, shrimp, etc.). The degree of deacetylation in commercial chitosans is in the range 60-100 %.

An object of the present invention is to provide a nanoparticle for delivery of RNA molecules, in particular RNA molecules that are capable of modulating gene expression or other biochemical activities in the cell.

Thus, in one aspect the invention provides a method of preparing a chitosan/RNA nanoparticle comprising the steps of:
a. Providing a chitosan solution
b. Providing an RNA solution
c. Mixing the solution of step a with the solution of step b
d. Incubating the solution of step c under conditions of complex formation such that chitosan/RNA nanoparticles form

### RNA solution

In a preferred embodiment of the invention, the RNA solution comprises RNA molecules of a length less than 100 nucleotides. In other embodiments, the RNA molecules are of a length less than 90 nucleotides, less than 80 nucleotides, less than 70 nucleotides, less than 60 nucleotides, less than 50 nucleotides, less than 40 nucleotides, less than 30 nucleotides and less than 22 nucleotides, respectively.

In a preferred embodiment of the invention, the RNA solution comprises RNA complexes of one or more RNA molecules, wherein the total number of nucleotides in the RNA complex is less than 100 nucleotides. In other embodiments, the total number of nucleotides is less than 90 nucleotides, less than 80 nucleotides, less than 70 nucleotides, less than 60 nucleotides, less than 50 nucleotides, less than 40 nucleotides and less than 30 nucleotides, respectively.

### RNA molecules and complexes

An RNA molecule as used herein is a stretch of ribonucleotides covalently linked. When two or more RNA molecules interact, e.g. through base pairing, an RNA complex is formed. Thus, as used herein an RNA complex comprises two or more RNA molecules. The RNA molecules of a complex may be identical or they may be different from each other. Preferably, they are capable of base pairing to each other such as to form a duplex over part of or the entire length of the RNA molecules.

RNA molecules may be modified by chemical modifications and may comprise deoxynucleotides, LNA nucleotides, phosphorothioates etc.

That RNA molecules of relative small size can form nanoparticles together with chitosan is surprising as chitosan has previously been used for formation of nanoparticles with plasmids that are much larger. A small plasmid is typically at least 5000 basepairs, i.e. 10.000 nucleotides. Moreover, plasmids are typically 100% double stranded.

In a preferred embodiment, one or more RNA molecules of the RNA solution are capable of mediating RNA interference to silence a target gene. Preferably, in this embodiment, the RNA is a small interfering RNA (siRNA) or a micro RNA (miRNA).

In another embodiment, the RNA is an aptamer capable of interacting specifically with an intracellular target, such as a protein.

### Initial cross linker

In another preferred embodiment of the method of preparing a chitosan/RNA nanoparticle, the chitosan does not does not comprise an initial crosslinker.

The term initial cross linker is used for a crosslinker that is added to chitosan to form a particle, before the RNA molecule is added. Chitosan/plasmid nanoparticles can be preformed using an initial crosslinker such as polyphosphate. Thus, it is believed that the structure and activity of a particle formed without an initial crosslinker differ from that of a particle formed with an initial crosslinker. In particular, the use of an initial crosslinker seems to imply that the RNA will be distributed at the surface of the preformed particle, whereas when using the RNA as crosslinker, the RNA will be distributed evenly through the particle. An even distribution is expected to a positive effect on the biostability of the RNA molecules of the nanoparticle, as they will be less accessible for RNases.

Moreover, the omission of the initial crosslinker provides are more facile method of preparation. Instead of a two-step method, where the particles are formed first and then the RNA is added, a one-step method is provided in which the RNA and chitosan is mixed to form nanoparticles directly.

Thus, in a preferred embodiment, the RNA functions as a crosslinker in the formation of a nanoparticle. In other words, the RNA is the form-active component.

### Concentrations

### RNA concentration

In one of embodiment of the method of preparing a chitosan/RNA nanoparticle, the RNA solution comprises RNA at a concentration selected from the group consisting of at least 5 µM, at least 10 µM, at least 20 µM, at least 30 µM, at least 40 µM, at least 50 µM, at least 60 µM, at least 70 µM, at least 80 µM, at least 90 µM and at least 100 µM.

### Chitosan concentration

In another embodiment, the chitosan solution comprises chitosan at a concentration from the group consisting of at least 50 µg/ml, at least 60 µg/ml, at least 70 µg/ml, at least 80 µg/ml, at least 90 µg/ml, at least 100 µg/ml, at least 110 µg/ml, at least 120 µg/ml, at least 130 µg/ml, at least 140 µg/ml, at least 150 µg/ml, at least 160 µg/ml, at least 170 µg/ml, at least 180 µg/ml, at least 190 µg/ml, at least 200 µg/ml, at least 250 µg/ml, at least 500 µg/ml, at least 750 µg/ml and at least 1000 µg/ml.

### Degree of deacetylation

Preferably, the chitosan has a relatively high degree of deacetylation. Thus, in one embodiment, the chitosan has a degree of deacetylation of selected from the group consisting of at least 60%, least 65%, least 70%, least 75%, least 80%, least 85% and at least 95%.

### Molecular weight of chitosan

The molecular weight of the chitosan is preferably more than 10 kDa. In another embodiment, the molecular weight is more than 50 kDa and even more preferred is a molecular weight of more than 80 kDa.

As outlined in the examples section, chitosan samples with a molecular weight above 80 kDa and a deacetylation degree above 50% are particular favourable.

### N:P ratio

The above mentioned parameters can all be used to control the characteristics of the formed nanoparticle. Another important parameter is the so-called N:P ratio, defined herein as the ratio of chitosan amino groups (N) to RNA phosphate groups (P).

In a preferred embodiment, the nanoparticle is formed at a N:P ratio larger than 50. Experiments document that increasing the N:P ratio, leads to larger particles. In other embodiments, the N:P ratio is selected from the group consisting of a N:P ratio larger than 60, larger than 70, larger than 80, larger than 90, larger than 100 and larger than 150.

In this preferred embodiment, wherein the N:P ratio is larger than 50, the RNA solution comprises RNA at a concentration lower than 100 µM, such as lower than 90 µM, lower than 80 µM, lower than 70 µM, lower than 60 µM, lower than 50 µM, lower than 40 µM, lower than 30 µM, lower than 20 µM, lower than 10 µM, lower than 5 µM or lower than 1 µM.

When employing a high N:P ratio and a low RNA concentration, nanoparticles can be formed that comprises loosely bound chitosan.

As the degree of loosely bound chitosan is dependent on both the concentration of RNA in the RNA solution and on the N:P ratio, the skilled worker will appreciate how to manipulate these parameters to create nanoparticles with loosely bound chitosan. E.g. a high RNA concentration of the RNA solution may be used, if also the N:P ratio is kept high, i.e. a high concentration of chitosan is used.

In one embodiment, the nanoparticle comprising loosely bound chitosan has a high N:P ratio.

A nanoparticle with loosely bound chitosan is of interest e.g. to improve mucosal delivery. Therefore, in one embodiment, the nanoparticle with loosely bound chitosan is for mucosal delivery.

A nanoparticle particle with a discrete character is of interest e.g. for systemic delivery. Such a particle can also be formed by controlling various parameters involved in the method of forming the nanoparticle. Particularly, a low N:P ratio favours the formation of a discrete nanoparticle. As mentioned above, the concentration of RNA and chitosan can be varied while maintaining a reasonably constant N:P ratio.

### Concentrated Method vs. N:P ratio

In this embodiment, the N:P ratio is lower than 70 such as but not limited to a N:P ratio lower than 60, lower than 50, lower than 40, lower than 30, lower than 20 or lower than 10, respectively.

In one embodiment, the nanoparticle of discrete character has a low N:P ratio.

In another embodiment, the concentration of the RNA solution is at least 100 µM, such as but no limited to at least 250 µM, at least 200 µM, at least 150 µM, at least 90 µM, at least 80 µM, at least 70 µM, at least 60 µM or at least 50 µM, respectively.

Using a high concentration of RNA in the RNA solution turns out to have several advantages. As outlined in the examples section, when the particles are formed using a RNA solution with a concentration of 250 µM, the particles are more discrete and monodispersed, as compared to particles formed using a pre-diluted RNA solution of 20 µM (as can be seen from the PDI measurements in table 3). Moreover, it surprisingly turns out that the nanoparticles formed using the concentrated RNA solution have a more specific effect, i.e. they do not give rise to any non-specific knockdown, which may be the case for particles formed using a RNA solution with a lower concentration of RNA.

Furthermore, using a high concentration of RNA in the RNA solution allows particle formation at a low pH such as ph 4.5, which in turn makes the particles more stable. Using a slightly higher pH of 5.5 is also possible. A pH of 5.5 may decrease detrimental effects of acetate buffer on cell viability.

Additionally, using a high concentration of RNA in the RNA solution means that the amount of RNA in particles increase, which decreases the amount of particle solution that has to be administered to a cell or organism.

In another embodiment, the chitosan concentration is less than 250 µg/ml.

In a particular preferred embodiment, the chitosan concentration is less than 250 µg/ml, while the RNA concentration is higher than 100 µM.

By controlling the concentrations of RNA and chitosan, and thereby the N:P ratio, also the size of the particles can be controlled, (as documented in the examples section. Thus, in one embodiment, the size of the particle is between 10 and 500 nM.

In another embodiment, the formed particles are discrete in form and have a polydispersity index lower than 0,4.

### Delivery

The nanoparticles may be used both for systemic delivery and for mucosal delivery.

Moreover, both nanoparticles of discrete form and nanoparticles with loosely bound chitosan may be used for aerosol delivery.

The droplets of the aerosol may be controlled by controlling the size of nanoparticles or parameters such as air and liquid pressures in aerosoliser devices. Thus, in one embodiment, the droplets of the aerosol are characterised in that they are smaller than 30 µm in diameters.

In addition to the method of preparing a nanoparticle, another aspect of the invention is the nanoparticle formed by the method.

### Method of treatment

Still another aspect is a method of treatment comprising the steps:
a. Providing a nanoparticle prepared by the method of the invention
b. Administrating said nanoparticle to a subject in need thereof

Since the RNA part of the nanoparticle may be used modulate the activity of a particular target, a nanoparticle comprising the RNA can be used for treatment. If for example the RNA is a siRNA, the sequence of the siRNA can be designed such as to sequence specifically target an mRNA. Thus, if it is known that a particular protein is causing disease or unwanted condition, the expression of the protein may be downregulated by using a siRNA that target the mRNA encoding the protein. Thereby, the disease or condition may be alleviated. This is very well known in the field of small interfering RNA and microRNAs. Also antisense RNA can be used to sequence specifically target an mRNA. Aptamers will typically have a protein as target, which however, also make them suited for therapeutics.

In one embodiment, the treatment comprises mucosal delivery of the nanoparticle.

In another more preferred embodiment, the mucosal delivery is selected from the group of delivery to the respiratory tract (upper and/or lower respiratory tract) oral delivery (oesophagus, stomach, small and large intestine) or genitourinary tract (e.g. anus, vagina) delivery.

Another aspect of the invention is a nanoparticle prepared by the above described method for use a medicament.

And still another aspect of the invention use of the nanoparticle prepared by the above described methods for the preparation of a medicament for treatment selected from the group of treatment of cancer, treatment of viral infections such as influenza, respiratory synthetic virus and bacterial infections e.g. tuberculosis and treatments of inflammatory conditions such as arthritis, chrones and hay fever.

### Research tool

Still another aspect of the invention is a method of delivering RNA into a mammal or cell comprising:
a. Providing a nanoparticle prepared by the method of the invention.
b. Subjecting said cell to the nanoparticle under conditions allowing entry of the RNA.

Such a method is for example of interest in basic research. Thus, the RNA may be a siRNA that is used to generate a knock-out of a target gene. Such experiments may be used to study the function of the target gene or to study interaction pathways. Such studies may be part of so-called target validation tests where the goal is to establish whether a particular gene or gene product might be a potential target for therapeutic intervention, e.g. by a siRNA. Therapeutic intervention may also be done by a small molecule, a peptide or a protein such an antibody.

The examples section demonstrates that nanoparticles of the invention can effectively deliver RNA into primary cells, which typically in the art is very difficult. Therefore, in one embodiment of the method of delivering RNA into a mammal or a cell, the cell is a primary cell.

In another embodiment, the cell is part of a mammal.

In still another embodiment, said mammal is non-human.

Still another aspect of the invention is a method of mediating RNA interference in a cell or organism comprising the steps of
a. Providing a nanoparticle prepared by the above described method
b. Subjecting said cell or organism to the nanoparticle under conditions allowing entry of the RNA.
c. Thereby mediating gene silencing of a gene corresponding to the RNA of the nanoparticle

### Figure legends:

Fig. 1
   Influence of chitosan and siRNA concentration on the formation of chitosan/siRNA nanoparticles.
   Atomic force microscopy images of chitosan/siRNA nanoparticles formed using 250 µg/ml chitosan; N:P 71 (Panel A), N:P 6 (Panel B) and chitosan 1 mg/ml; N:P 285 (Panel C), N:P 23 (Panel D). Inserts show representative of large particles within image. (Large image 6x6 µm, scale bar 600 nm; Insert 1x1µm, scale bar 250 nm).
Fig. 2
   Live cellular uptake of chitosan/siRNA nanoparticles into NIH 3T3 cells. Fluorescence microscopy was used to visualize cellular uptake and translocation of Cy5-labeled siRNA (100 nM/well) within chitosan nanoparticles (A, 1 h; B, 4 h; and C, 24 h) Images show fluorescent overlay of siRNA (red Cy5-labeled) and nuclei (blue Hoechst-labeled) adjacent to phase contrast image (scale bar, 10 Am).
Fig. 3
   Nanoparticle-mediated RNA interference in the EGFP-H1299 cell line.
   (A) EGFP-H1299 cell line transfection with chitosan/EGFP-specific siRNA nanoparticles (NP) or TransIT/TKO/EGFP-specific siRNA (TKO) for 4 h (50 nM siRNA/well) with or without chloroquine (10 µM/well). The decrease in EGFP mean fluorescence intensity, detected by flow cytometry at 48 h post-transfection, was used as the measure of EGFP knockdown (normalized to % control untreated EGFP cells, error bars represent +/- SD). Chitosan/EGFP-mismatch siRNA nanoparticles (NP) or TransIT/TKO/EGFP-mismatch siRNA (TKO) controls are also shown. (B) EGFP-H1299 cell viability after treatment with same formulations used in transfection study (normalized to % control untreated cells) (error bars represent +/- SD).
Fig. 4
   Nanoparticle-mediated RNA interference in primary macrophages.
   EGFP knockdown in macrophages isolated from EGFP transgenic mice is shown. (A) Fluorescent micrograph showing chitosan nanoparticle uptake after 4 h (Panel A, light image; Panel B, Cy-5 labelled siRNA (red)). Untreated EGFP macrophages (Panel C, light image; Panel D, cellular EGFP fluorescence (green). Macrophage cellular fluorescence 24 h post-transfection with EGFP-specific siRNA (100 nM/well for 4 h) with TransIT-TKO (TKO) (Panel E-F) and chitosan nanoparticles (NP) (Panel G-H). (B) Flow cytometric analysis of macrophage EGFP fluorescence. In addition, analysis of cells transfected with 200 nM siRNA is presented. Peritoneal primary macrophages isolated from non-green mice were used as an EGFP negative.
Fig. 5
   Pulmonary RNA interference in the transgenic EGFP mouse.
   The number of EGFP expressing endothelial cells of the bronchioles, was counted by fluorescence microscopy, in 3 µm lung sections taken from EGFP mice (n=2-3) nasally dosed with chitosan/EGFP-siRNA nanoparticles (30 µg siRNA per day for 5 days) (two separate experiments presented). The representative images show EGFP fluorescence (green) and DAPI-stained nuclei (blue) overlay within the bronchiole region of control mice (Panel A) and mice treated with chitosan/EGFP-specific siRNA nanoparticles (Panel B). The numbers of EGFP positive cells expressed as a percentage (%) of 200 epithelial cells counted are presented in C (experiment 1, untreated mice used as control) and total number of EGFP positive cells (#) from whole left lung are presented in D (experiment 2, EGFP-mismatch used as control).
Fig. 6
   Nanoparticle protection to serum-induced siRNA degradation.
   The electrophoretic migration of chitosan/siRNA (N:P 71 and 6) in the presence of 10% fetal calf serum (FCS) (4 h, 37°C) +/- poly(aspartic acid) (PAA) (30min, 37°C) was visualised using a 10% polyacrylamide gel (150-230V, 2 h). Lane 1, naked siRNA; Lane 2, naked siRNA in 10% FCS; Lane 3, nanoparticles (N:P 71); Lane 4, nanoparticles (N:P 71) in 10% FCS; Lane 5, nanoparticles (N:P 71) in FCS + PAA; Lane 6, nanoparticles (N:P 6), Lane 7, nanoparticles (N:P 6) in FCS; Lane 8, nanoparticles in FCS + PAA. Nanoparticles were loaded at the same siRNA concentration.
Fig. 7
   Evaluation of cellular cytoxicity of chitosan/siRNA nanoparticles.
   Chitosan/siRNA nanoparticles formed using 250 µg/ml chitosan; N:P 71, (A), N:P 6 (B) and 1 mg/ml chitosan; N:P 285 (C) N:P 23 (D) were added to NIH 3T3 cells at 50nM siRNA concentration. After 24 h the cellular uptake of CellTitre Aqueous one solution reagent was used to measure cell viability (normalized to % control untreated cells) (error bars represent +/- SD).
Fig. 8
   Knockdown of BCR/ABL-1 leukemia fusion protein in K562 (Ph+) cell line.
   K562 (Ph+) cells were transfected with 50nM BCR/ABL-specific siRNA within chitosan nanoparticles for 24 h. 48 h post-transfection the amount of BCR and BCR/ABL protein was detected by western blotting using a rabbit polyclonal antibody against the N-terminus of BCR. (A) BCR/ABL-1 mRNA target sequence (B) BCR/ABL-1 specific siRNA (C) Lane 1, untreated cell line; Lane 2, non-specific siRNA/chitosan nanoparticles; lane 3, BCR/ABL-1-specific siRNA/chitosan nanoparticles. The non-targeted transcript hnRNP C1 is used as an internal control. Histogram expresses detected protein levels as percentage BCR/ABL-1 knockdown
Fig. 9
   Effect of chitosan M_{w} and DD on the size (A), zeta potential (B) of chitosan/siRNA formulations.
Fig. 10.
   AFM images of chitosan/siRNA formulations formed with different chitosan samples at N:P ratio of 50:1 on mica surface.
Fig. 11
   Effect of chitosan M_{w} and DD on the complex stability of chitosan/siRNA formulaitons.
   Lane 1: siRNA-eGFP; Lane 2: C9-95/GFP; Lane 3: C12-77/GFP; Lane 4: C65-78/GFP; Lane 5: C114-84/GFP; Lane 6: C170-84/GFP; Lane 7: C173-54/GFP; Lanes 8: C9-95/GFP + poly(aspartic acid) (PAA); Lanes 9: C12-77/GFP + PAA; Lanes 10: C65-78/GFP + PAA; Lanes 11: C114-84/GFP + PAA; Lanes 12: C170-84/GFP + PAA; Lanes 13: C173-54/GFP + PAA.
Fig. 12
   Gene silencing efficiency and cytotoxicity of chitosan/siRNA nanoparticles at N:P raito of 50:1.
   (A) *in vitro* transfection of nanoparticles prepared with 6 chitosan samples of different M_{w} and DD; (B) cytotoxicity assay of nanoparticles prepared with 6 chitosan samples of different M_{w} and DD.
   Untransfected cells (control), nanoparticles prepared with commercial transfection reagent, Mirus TransIT TKO^{®}, is included as positive control.
Fig. 13
   Gene silencing efficiency and cytotoxicity of chitosan/siRNA nanoparticles at N:P raito of 150:1.
   (A) *in vitro* transfection of nanoparticles prepared with 6 chitosan samples of different M_{w} and DD; (B) cytotoxicity assay of nanoparticles prepared with 6 chitosan samples of different M_{w} and DD.
   Non-transfected cells (Control), nanoparticles prepared with commercial transfection reagent, Mirus TransIT TKO^{®}, is included as positive control.
Fig. 14
   Effect of N:P ratios on the complex stability of nanoparticles formulated with chitosan (C170-84).
   Lane 1: siRNA-eGFP; Lane 2: 2:1; Lane 3: 10:1; Lane 4: 50:1; Lane 5: 150:1
Fig 15
   AFM showing morphological comparison of nanoparticles formed using direct additions of siRNA (concentrated method) or pre-diluted siRNA (diluted method)

### Examples

### Example 1

### Results

### Physicochemical characterisation

All chitosan/siRNA formulations were found to have a hydrodynamic radius lower than 350 nm by PCS analysis (Table 1).

**Table 1: Photon correlation spectroscopy of chitosan/siRNA nanoparticles**

| Formulation | Size (nm)/(Polydispersity Index) | | | | Zeta Potential (mV) |
|---|---|---|---|---|---|
| | Absence of salt | Presence of salt^{a} | | | |
| | | 10 min | 1 h | 24 h | |
| | | | | | |
| A (N:P71) | 181.6 (0.22) | 173.2 (0.23) | 175.9 (0.23) | 176.4 (0.22) | 26.8 (1.06) |
| B (N:P6) | 223.6 (0.19) | 219.7 (0.21) | 232.3 (0.23) | 228.1 (0.20) | 18.8(2.11) |
| C (N:P 285) | 139.0 (0.48) | 132.8 (0.49) | 138.0 (0.52) | 136.3 (0.51) | 29.5 (0,50) |
| D (N:P 23) | 328.0 (0.24) | 310.0 (0.23) | 314.2 (0.24) | 319.4 (0.23) | 31.1 (0.74) |

| | | | | | |
|---|---|---|---|---|---|
| A, B 250 µg/ml chitosan (Mw 114 kDa) and C, D 1 mg/ml chitosan (Mw 114 kDa) ^{a}Incubation in 0.15M NaCl before readings taken; Size and ζ potential average of 3 determinations, zeta potential SD in brackets. | | | | | |

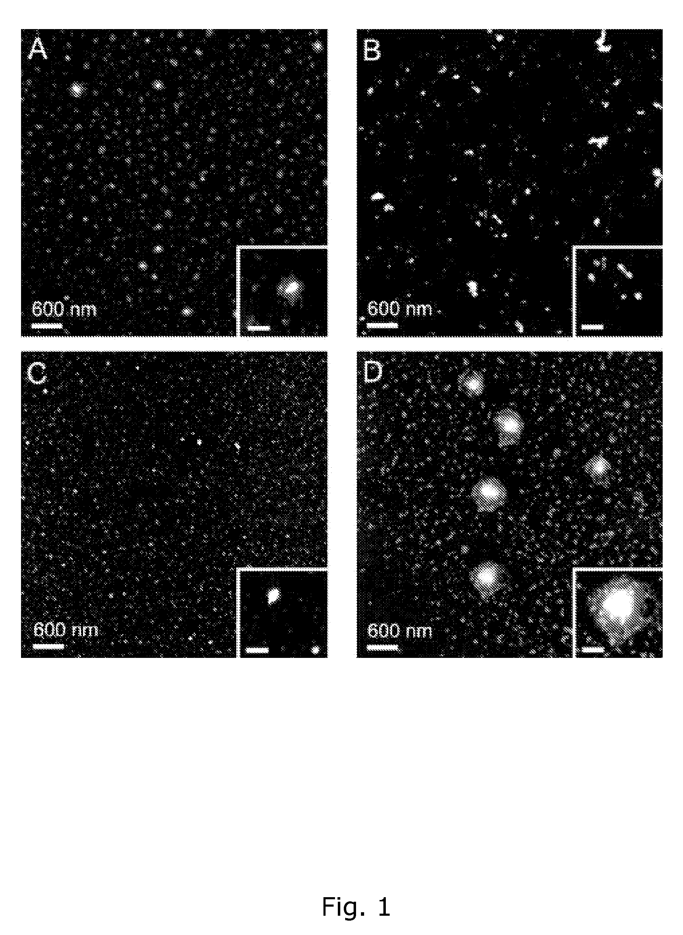

The size of nanoparticle formation was dependent on the N:P ratio (defined as the ratio of chitosan amino groups (N) to RNA phosphate groups (P)) with increase in size at lower N:P. At low chitosan concentrations (250 µg/ml), nanoparticles formed at N:P 71 (formulation A) measured 181.6 nm but increased to 223.6 nm at N:P 6 (formulation B). Similarly, at high chitosan concentrations (1 mg/ml) the nanoparticle hydrodynamic radius increased from 139 nm at N:P 285 (formulation C) to 328 nm at N:P 23 (formulation D). This suggests more siRNA bond-forming bridges between chitosan chains at increased siRNA levels leading to greater chitosan incorporation and possible inter-particle aggregation. The observation that the saturation level of chitosan was reached in all formulations (verified by chitosan detection in particle filtrate, data not shown) confirms siRNA as the nanoparticle size determinant. The addition of salt over 24 h had no substantial effect on the hydrodynamic radius of the formulations studied. This may be due to repulsive forces between the particles being significantly stronger than hydrophobic self-association following neutralisation of charge. Zeta potential measurements showed all formulations had a net positive charge greater than 18 mV (A, 26.8, B, 18.8, C, 29.5 and D 31.1), reflecting excess chitosan at N:P greater than 1.0 (confirmed by acid urea gel analysis of chitosan, data not shown). The zeta potential decrease (18.8 mV) in B formed at lower levels of chitosan (250 µg/ml) and high siRNA reflects a greater chitosan incorporation and reduction in free material. Atomic force microscopy revealed a polydispersed distribution of predominantly spherical nanoparticles (Fig. 1). A population of extremely small discrete complexes (54-78 nm) were apparent in all the formulations (Fig. 1A-D). Large structures greater than 250 nm (size dependent on chitosan and siRNA concentration) were also observed, suggesting recruitment of the smaller structures into aggregates (Fig. 1 inserts A-D). Complexes of 600 nm average length were formed with the highest chitosan and siRNA concentrations (Fig. 1 insert D).

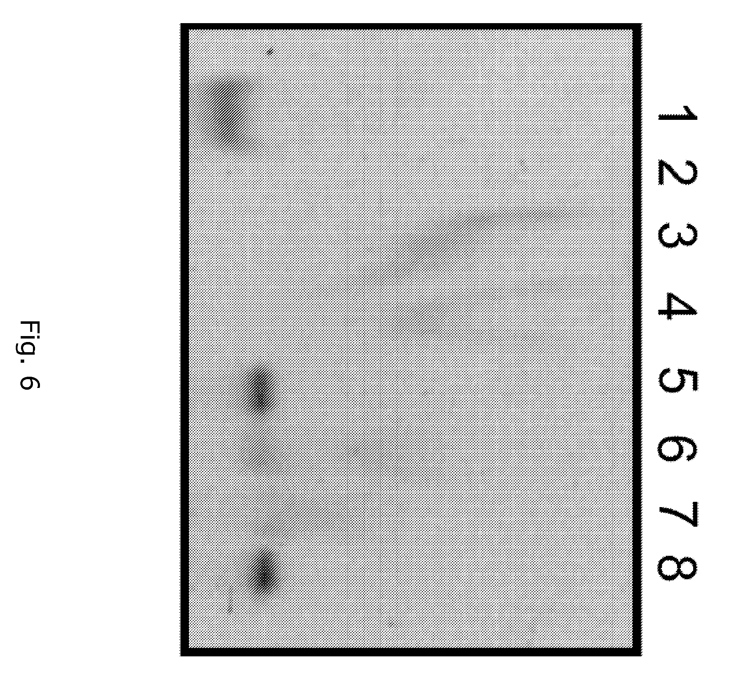

The electrophoretic migration of siRNA was used to investigate complex formation and nanoparticle stability (figure 6). Retardation of RNA within the gel showed complex formation between chitosan and siRNA at N:P 71 and 6. Interestingly, slower migration was observed at higher N:P ratio suggesting the influence of increased amounts of free excess chitosan on particle stability. The requirement of polyanionic displacement with PAA for RNA release from the nanoparticles confirmed electrostatic chitosan/siRNA interactions. Moreover, intact siRNA was maintained after release from serum-incubated nanoparticles (N:P 71 and 6) whereas non-formulated naked RNA was degraded. This suggests that the chitosan effectively protects the siRNA against nuclease breakdown.

### Cellular studies

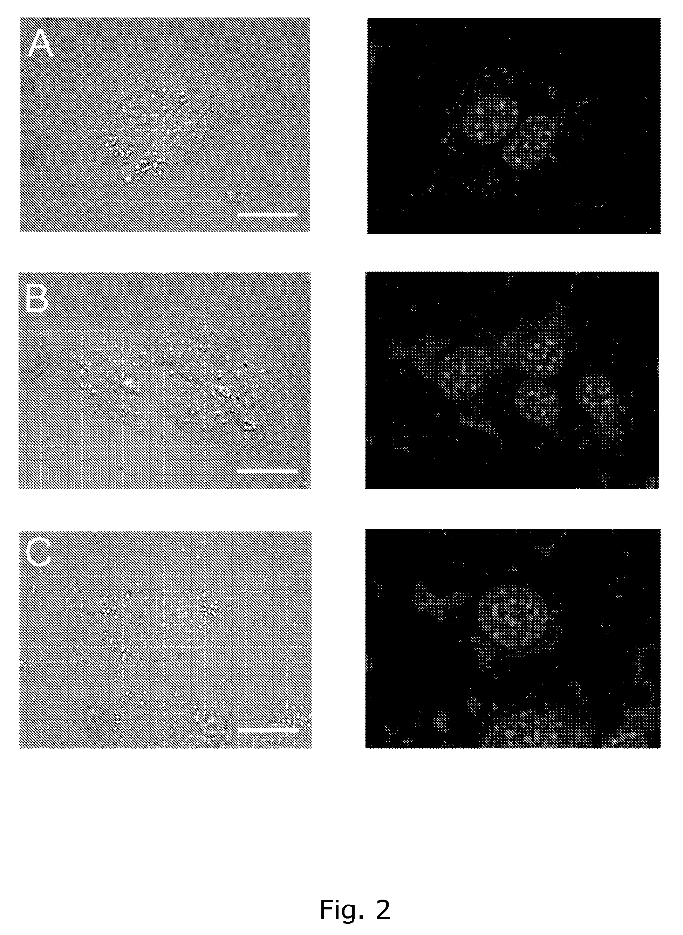

Intracellular trafficking of nanoparticles containing Cy5-labeled siRNA (N:P 36) was investigated in live NIH 3T3 cells using semi-confocal epifluorescence microscopy (Fig. 2). Punctuate fluorescence within the apical regions of cells, defining cellular borders, was visible after 1 h (Fig. 2A). At 4 h, fluorescence could be seen within all areas of the cell including nuclear regions (Fig. 2B). The fluorescence was more diffuse in appearance compared to images taken at 1 h, suggesting possible particle dissociation and siRNA release. In contrast, limited fluorescence was observed after 4 h in cells transfected with Cy5-labelled siRNA with *TransIT-TKO* (Fig. 2D). Fluorescence was found throughout the cell after 24 h with both nanoparticles and *Trans*IT-TKO (Fig. 2C and 2E, respectively).

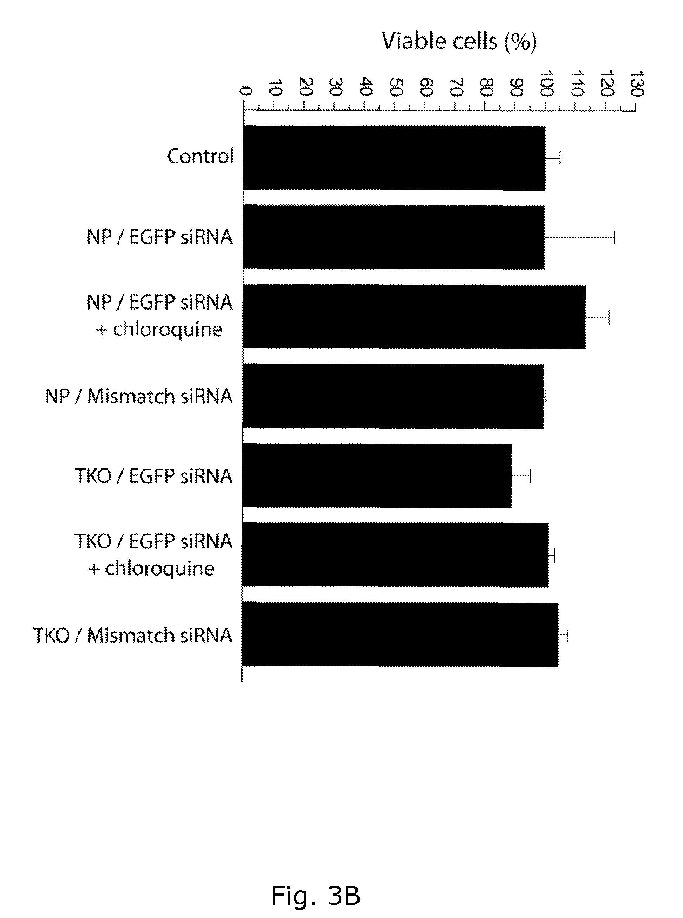

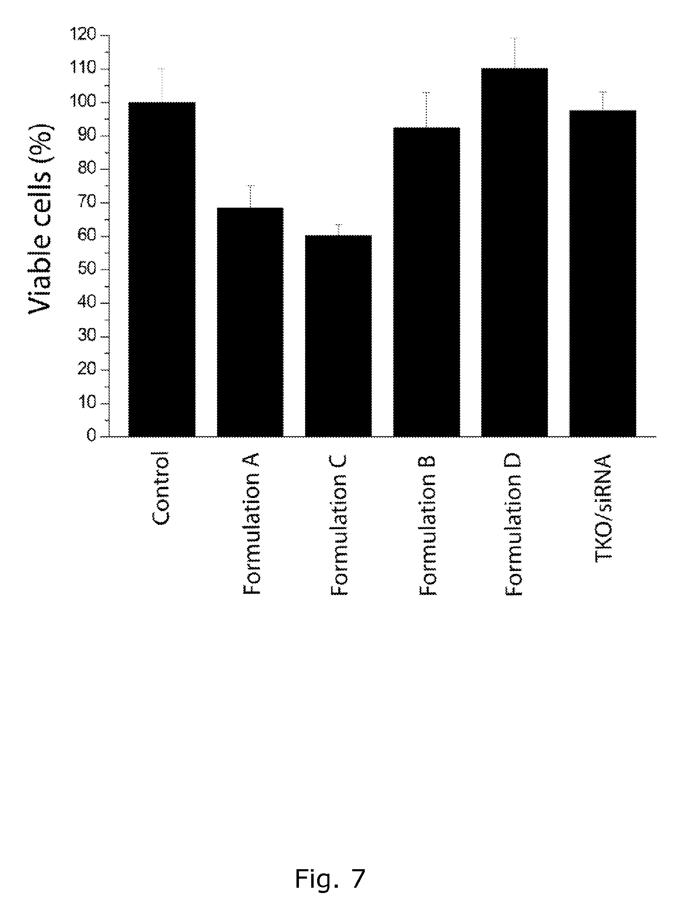

Cellular cytoxicity of chitosan and *Trans*IT-TKO formulated particles were evaluated in NIH 3T3 cells using a tetrazolium-based viability assay, figure 7). Chitosan nanoparticles formed at N:P 71 (formulation A) and 285 (formulation C) reduced cell viability (31.5 % and 39.8 %, respectively) when added at a concentration of 50 nM siRNA/well. In contrast, and in accordance with *Trans*IT-TKO treated cells, cell viability was maintained with chitosan nanoparticles formulated at low N:P (6 and 23) (formulation B and D, respectively) using the same siRNA concentration. This suggests that excess chitosan in nanoparticles formed at high N:P may decreases cell viability related to the possible toxicity associated with high molecular weight chitosan that has been reported previously. This data provided information and guidelines for the development of non-cytotoxic formulations (below N:P 71) taken forward into later transfection studies (refer to Fig. 3B).

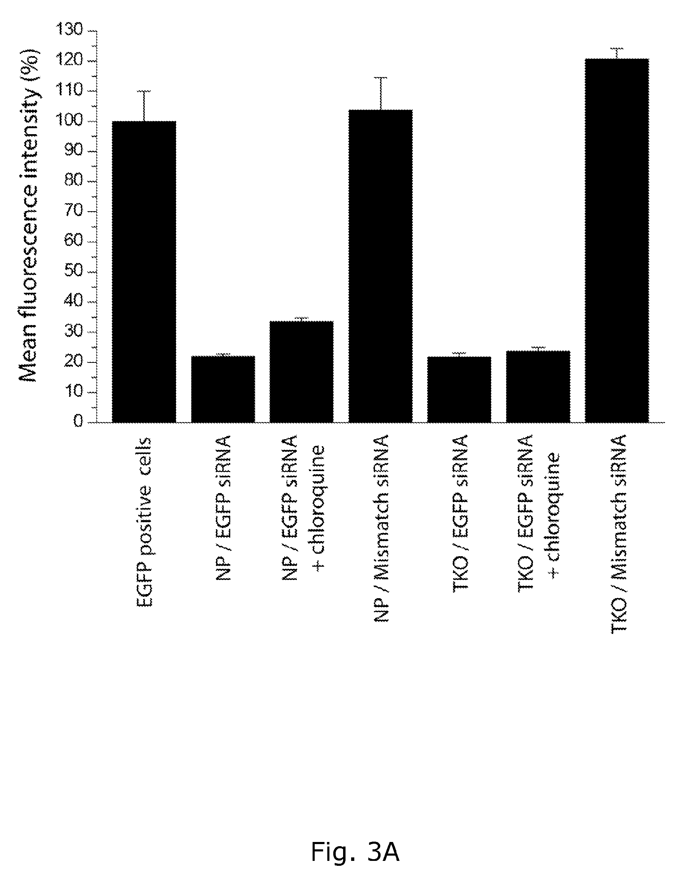

RNA interference of endogenous protein expression was investigated in the H1299 cell line containing a stably integrated EGFP expression cassette and in primary macrophages from an EGFP transgenic mouse using nanoparticles containing EGFP-siRNA. The decrease in EGFP mean fluorescence intensity, detected by flow cytometry at 48-h post transfection, was used as the measure of EGFP knockdown. Figure 3A shows significant EGFP knockdown (77.9 %) in H1299 cells 48 h post-transfection after an initial 4 h chitosan nanoparticle (N:P 57) transfection; levels comparable to *TransIT-TKO* (78.0 %). Knockdown was not significantly influenced in the presence of the endosomolytic agent chloroquine (66.4 %) suggesting the capability of the nanoparticle system to escape endosomal compartments prior to RNA interaction with target mRNA. Chitosan nanoparticles containing EGFP-mismatch siRNA (4 base pair mismatch) showed no EGFP knockdown, confirming knockdown specificity. Cellular viability in H1299 cells was maintained after addition of the different formulations used for transfection (Fig. 3B), dismissing the likelihood of toxicity effects.

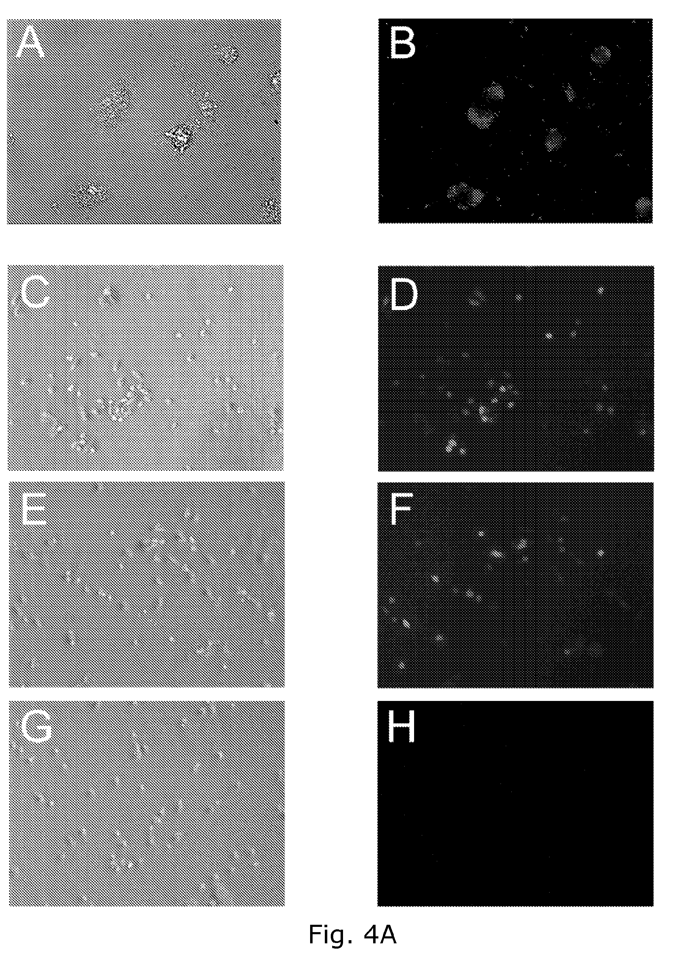

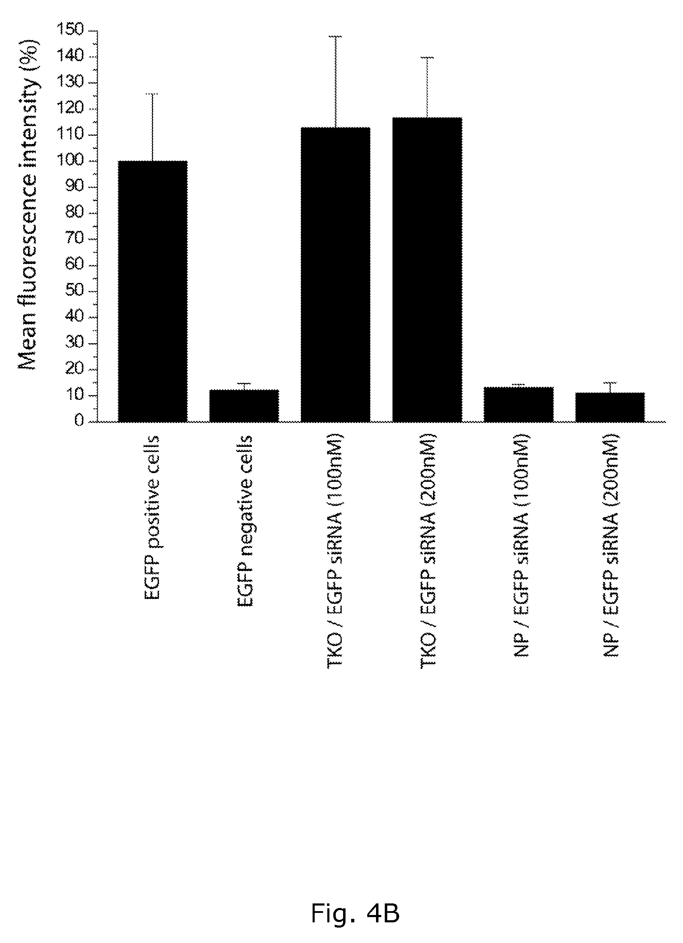

The ability of the nanoparticle system (N:P 36) to mediate interference in primary cells was evaluated in peritoneal macrophages isolated from transgenic EGFP mice (Fig. 4A and 4B). Macrophages, visualised by fluorescence microscopy, showed almost complete loss of EGFP fluorescence following 24 h post-transfection with chitosan nanoparticles (Fig. 4A, Panel G-H) compared to untreated control (Fig. 4A, Panel C-D), indicating significant knockdown. In contrast, cells transfected with *Trans*IT-TKO maintained EGFP expression (Fig. 4A, Panel E-F). This trend was verified using flow cytometric measurements of samples (Fig. 4B). High levels of EGFP knockdown was found in cells treated with nanoparticles (86.9 %) compared to *TransIT-TKO* (no knockdown detected) (Fig. 4B). This correlates with the avid uptake into macrophages shown by nanoparticles containing CY5-labelled siRNA (Fig. 4A, Panel A-B).

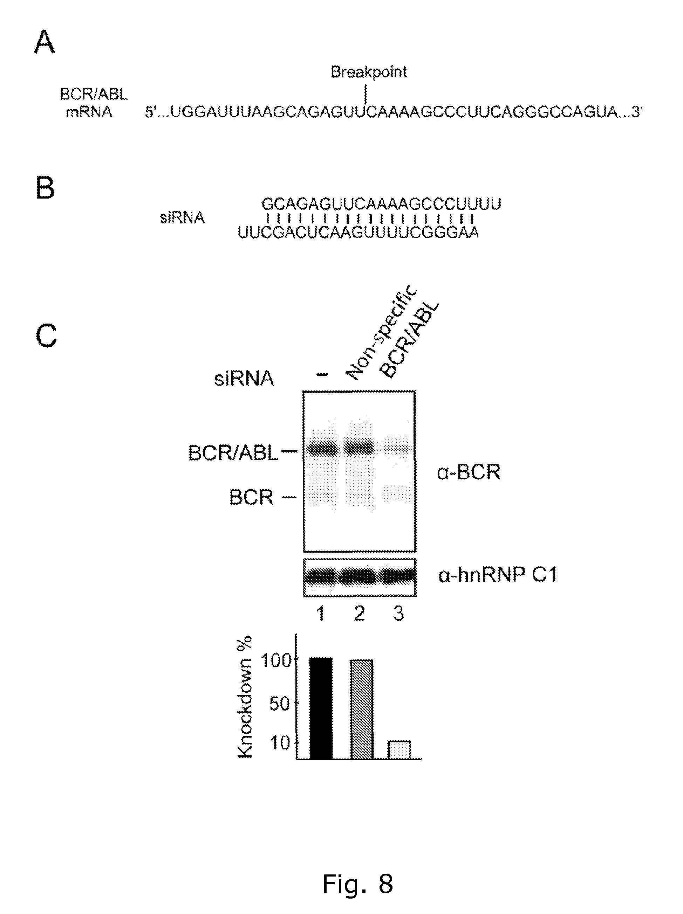

We tested the ability of the nanoparticles to knockdown expression of the BCR/ABL-1 oncogene, found in chronic myelogenous leukemia, to demonstrate the therapeutic potential of the chitosan system (Fig. 8). The BCR/ABL-1 expressing cell line K562 was transiently transfected with nanoparticles (N:P 57) complexed with either a fusion-specific or a non-specific siRNA. Western blotting analysis using an antibody recognising the N-terminal part of BCR demonstrated ~90% BCR/ABL-1 knockdown in cells transfected with nanoparticles containing BCR/ABL-1-specific siRNA.

### Pulmonary RNA interference

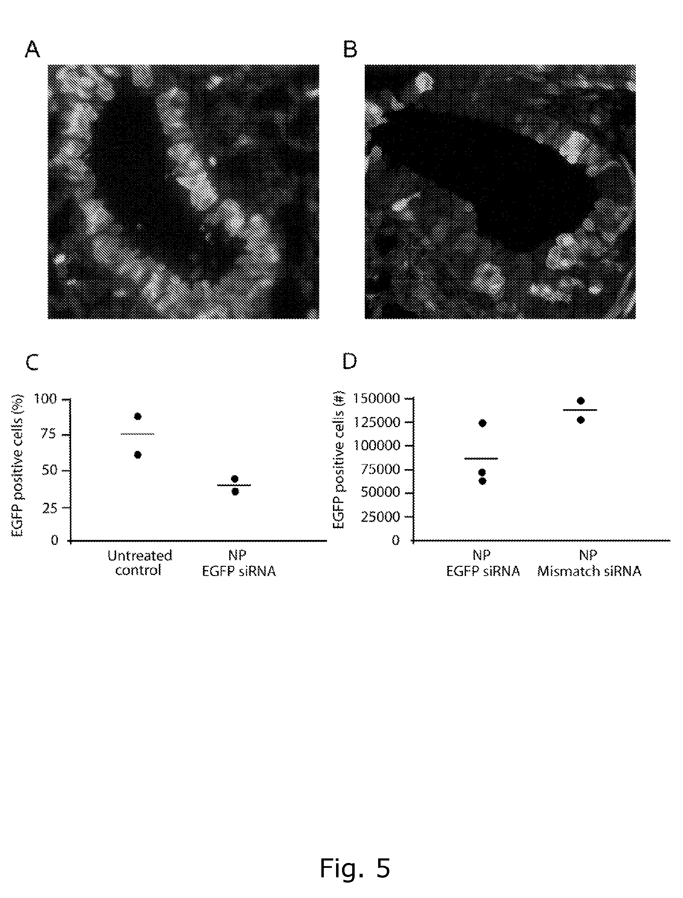

A transgenic EGFP mouse model was used to investigate the ability of chitosan-based systems to mediate EGFP knockdown following nasal administration. None of the mice appeared to have any adverse effects from daily nasal administration of chitosan nanoparticles (N:P 6) over the 5 day period. The mice were subjected to whole body perfusion fixation following siRNA treatment and lung sections were investigated by fluorescence microscopy. In two separate experiments, mice dosed with nanoparticles containing EGFP siRNA, clearly showed reduced numbers of EGFP expressing epithelial cells in the bronchioles (43 % compared to untreated mice control (experiment 1, Fig. 5C) and 37 % compared to EGFP-mismatch control (experiment 2, Fig. 5D). Representative images of control (Fig. 5A) and knockdown (Fig. 5B) are shown. This phenomenon was observed both in lower and upper regions of the lung. The number of green cells as a percentage of 200 cells per tissue section (experiment 1, Fig 5C) or total numbers in the whole left lung (experiment 2, Fig. 5D) was calculated by stereological counting. The presence of DAPI-stained nuclei demonstrated an intact epithelial bronchiole border in control and nanoparticle dosed mice following treatment and histological processing.

### Discussion

We introduce a novel chitosan-based siRNA delivery system for RNA interference protocols, the rational based on the exploitation of chitosan properties to allow mucosal delivery.

Self-assembly of siRNA into nanoparticles were formed at low (250 µg/ml) and high (1 mg/ml) chitosan concentrations with the RNA sufficient in length (21 nucleotides) to meet the 6-10 salt bond requirement for the formation of a corporative system between polyelectrolyte species. AFM analysis revealed a predominate population of small discrete particles in the 50nm size range and a larger subpopulation ranging from 200-500nm, suggesting inter-particle aggregation following initial entropy-driven particle formation. This was confirmed by PCS measurements, which showed small and large hydrodynamic radius distributed around a mean of 180-330 nm. In accordance with drug delivery requirements, release of structurally intact siRNA from the nanoparticles was demonstrated by electrophoresis; an essential prerequisite for nanocarrier-mediated RNA gene silencing.

As much as 77.9 % specific reduction of EGFP fluorescence was measured in an EGFP-H1299 human lung carcinoma cell line transfected for 4 h with chitosan nanoparticles. These levels were similar to knockdown observed in cells transfected for the longer period of 24 h with TransIT-TKO transfection reagent. The relatively short transfection time required when using chitosan is probably a result of the rapid siRNA accumulation observed in NIH 3T3 cells within a 4 h period using chitosan nanoparticles. The cellular uptake properties of chitosan nanoparticles can be attributed to the small particle size and excess positive charge that facilitate interaction with cellular membranes. Transfection in the presence of the endosomolytic agent chloroquine did not increase RNA interference with the chitosan system, indicating an endosomal escape mechanism for the chitosan-based system. In support of this, a diffuse cellular distribution of Cy5-labelled siRNA was visualized throughout the whole cytoplasm with no evidence of compartmentalization in NIH 3T3 cells.

The therapeutic potential of chitosan nanoparticles for knockdown of disease-related proteins was demonstrated in K562 cells endogenously expressing BCR/ABL-1 protein. Transfection with a single treatment of chitosan nanoparticles containing breakpoint siRNA resulted in ~90% allele specific knockdown. The chitosan system was the only chemical transfection agent, including commercial alternatives that successfully showed BCR/ABL-1 knockdown in this suspension cell line in our experiments (data not shown). In comparison to other polycation-based polyethylenimine systems that have reported knockdown in adherent cell lines, our work shows efficient knockdown in both adherent and suspension cells.

Macrophages are important cells in inflammatory and viral disease and consequently a target for RNA interference therapies. In our experiments, peritoneal macrophages isolated from transgenic EGFP mice were used to evaluate chitosan-based knockdown in primary cells and to provide an appropriate pre-*in vivo* model. We showed 89.3 % reduced EGFP levels (compared to non-treated cells) within 1 day following a single 4 h treatment with chitosan nanoparticles, which correlates with rapid uptake of chitosan nanoparticles into these cells in less than 4 h. In contrast, *Trans*IT-TKO transfection gave no significant EGFP knockdown under the same conditions. To our knowledge, this is the first report showing knockdown in primary macrophages with a polycation-based system.

The nasal route offers a non-invasive alternative to the systemic administration of siRNA therapeutics. It provides direct access to respiratory tissue and a migration pathway to systemic sites with avoidance of first-pass hepatic clearance. Our strategy is to exploit the mucoadhesive and permeation properties of chitosan for effective delivery and RNA interference at respiratory sites as an approach to treat pulmonary disease. The transgenic EGFP mouse used in our experiments showed significant EGFP knockdown (43 % compared to untreated control and 37 % compared to EGFP-mismatch) within bronchiole epithelial cells following a nasal dose of nanoparticles containing EGFP-specific siRNA. This is the first documentation to our knowledge of knockdown within this cell type after nasal administration.

### Materials and Methods

### siRNA sequences

EGFP-specific siRNA duplex containing the sequence: sense, 5'-GACGUAAACGGCCACAAGUUC-3', antisense, 3'-CGCUGCAUUUGCCGGUGUUCA-5' and EGFP-mismatch sense, 5'-GACGUUAGACUGACAAGUUC-3', antisense, 3'-CGCUGAAUCUGACCUGUGGUUCA-5' were used for nanoparticle characterisation studies and EGFP interference work. EGFP siRNA containing a fluorescent Cy5 labelled 5'sense strand was used for cellular uptake studies. BCR/ABL-1 siRNA target sequence: AAGCAGAGUUCAAAAGCCCUU, control target sequence: AAGGAGAAUAGCAGAAUGCAU were used for leukemia translocation protein knockdown.

Formation of chitosan/siRNA nanoparticles. Chitosan (114 kDa) was dissolved in sodium acetate buffer (0.2M NaAc, pH 4.5) to obtain a 1 mg/ml or 250µg/ml working solution. 20 µl of siRNA (20-250 µm range) was added to 1 ml of filtered chitosan whilst stirring and left for 1 hour. To calculate specific N: P ratios (defined as the molar ratio of chitosan amino groups/RNA phosphate groups) a mass-per-phosphate of 325 Da was used for RNA and mass-per-charge of chitosan 167.88 (84% deacetylation).

Photon correlation spectroscopy and determination of surface charge on chitosan/siRNA nanoparticles. The hydrodynamic size of Chitosan/siRNA complexes were determined by photon correlation spectroscopy (PCS). PCS was performed at 25°C in sodium acetate buffer in triplicate with sampling time and analysis set to automatic. Particle size is presented as the z-average of three measurements +/- SD. To investigate the effect of salt on hydrodynamic size, sodium chloride (final concentration 150 mM) was added to the particle solution for set time points before remeasurement. Surface charge was measured by determination of zeta potential using a Zetasizer Nano ZS in sodium acetate buffer.

Determination of nanoparticle morphology using Atomic Force Microscopy. Chitosan/siRNA nanoparticles were diluted 1/10 using 0.2 µm filtered sodium acetate buffer. A sample volume of 15 µl was immobilised onto freshly cleaved mica. The samples were purged with N2 and imaged in Tapping Mode under ambient conditions on a nanoscope IIIA (Digital Instruments) using NSG01 (NT-MDT) cantilevers with a tip radius less than 10 nm. Several images were obtained for each sample, ensuring data reproducibility.

Cellular uptake of Cy5-labeled siRNA chitosan nanoparticles. NIH 3T3 cells or mouse peritoneal macrophages were cultured in 35 mm dishes with glass cover slip bottoms and transfected with 100 nM Cy5-labeled sense strand EGFP siRNA using chitosan nanoparticles (NP 36) or *Trans*IT-TKO transfection reagent. The cells were transfected with the nanoparticles in DMEM serum-free media for 1-4 hours after which 10% serum was added. *Trans*IT-TKO transfection was done in serum-containing medium according to Mirus protocol. Following transfection cells were subjected to Hoechst staining in order to visualize nuclei. Uptake of duplex siRNA was monitored by a Zeiss semi-confocal epifluorescence microscope.

RNA interference in EGFP expressed human cell line and primary murine macrophages. The human lung cancer cell line H1299 produced to stably express EGFP (EGFP half-life 2 h) was a gift from Dr Anne Chauchereau (CNRS, Villejuif, France). Cells were plated on multiwell 24-plates (10⁵cells/well) in RPMI media (containing 10% fetal bovine serum (FBS), 5% penicillin/streptomycin, and 418 selection factor) 24 h prior to transfection. The media was removed and replaced with 250µl fresh media (with or without FBS). The cells were transfected with chitosan/siRNA nanoparticles or *Trans*IT-TKO transfection reagent at 50 nM siRNA (EGFP-specific or EGFP-mismatch) per well in the presence or absence of chloroquine (10um per well, added 10 min prior to addition of siRNA). After 4 h, the media was replaced with 0.5ml fresh media containing 10% FBS. The cells were left for 48 h and then removed using a standard trypsin protocol and resuspended in PBS containing 1% paraformaldehyde. The EGFP cell fluorescence was measured using a Becton Dickenson FACSCalibur flow cytometer. A histogram plot with log green fluorescence intensity on the x-axis and cell number on the y-axis is used to define median fluorescence intensity of the main cell population defined by scatter properties (FSC, SSC, not shown).

Adult EGFP-transgenic mice (C57BL/6-Yg (ACTbEGFP) 1Osb/J) (Jackson Laboratories, Maine, USA) were killed by cervical dislocation and injected intraperitoneally with 5 ml of MEM media containing 20% FBS. The abdomen was agitated gently, the peritoneum exposed and breached, and the media removed using a syringe. The media was centrifuged (2500 rpm for 10 min) and pellet resuspended in MEM media containing 50% FBS. The suspension was plated on a multiwell 12-well plate. The macrophages were allowed to adhere for 2 h before media (containing non-adherent cells) was removed. Fresh media containing 5% Penicillin/Streptomycin was then added the cells. After 40 h the media was removed and replaced with MEM media without FBS and chitosan/siRNA nanoparticles or *Trans*IT-TKO/siRNA added. After 4 h, media was removed and replaced with fresh media containing 10% FBS. After 24 h the cells were processed and analysed for EGFP fluorescence using a BD FACSCalibur flow cytometer flow cytometer or visualised using a Zeiss semi-confocal epifluorescence microscope. Untreated macrophages isolated from C57BL/6J mice were used as non-fluorescent controls.

Pulmonary RNA interference in EGFP mouse. EGFP-transgenic mice (C57BL/6-Yg(ACTbEGFP)1Osb/J) used for *in vivo* siRNA studies were housed in SFP conditions under strict veterinary supervision at Pipeline Biotech A/S (Trige, Denmark). Chitosan/siRNA particles were concentrated to 1 mg/ml siRNA using VivaSpin20 centrufugal concentrators (MW cut-off 100 KDa, Vivascience) prior to dosing. In two different experiments, a total of 30 µl of particles were administered intranasally (15 µl per nostril) each day over 5 consecutive days to the EGFP mice (non-dosed C57BL/6J mice were used as control in experiment 1 and EGFP-mismatch as control in experiment 2). At day 6 mice were anesthetised with an injection of 0.14 ml zoletilmix/torbugesic mix and perfusion fixated manually with 4% formaldehyde phosphate buffered solution (Volusol, VWR International). Lungs were harvested, paraffin embedded and cut exhaustively in 3 µm sections and every 100th section together with the next was sampled. Sections were transferred into DAPI (Sigma, St. Louis, USA) solution for counterstaining and wet mounted on a super frost slide. Slides were analysed in a fluorescent microscope (Olympus BX 51, Tokyo, Japan) with a UV/GFP filter, a 20X objective, a mounted digital camera (Olympus DP-70), and a motorized stage in conjunction with CAST software (Visiopharm, Copenhagen Denmark). The number of EGFP expressing epithelial bronchial cells was counted by a physical fractionator.

### Example 2

### Materials and methods

### Materials

The chitosan samples used in this study were all prepared using standard methods (Vårum KM et al, 1994; Ottøy MH et al., 1996;Hirano S et al 1976; Muzzarelli RAA, Rocchetti R 1985; Khan et al., 2002) siRNA-EGFP duplex (21 bp), sense sequence: 5'-GACGUAAACGGCCACAAGUUC-3', antisense sequence: 3'-CGCUGCAUUUGCCGGUGUUCA-5') and four-base mismatch siRNA-EGFP (21 bp), sense sequence: 5'-GACUUAGACUGACACAAGUUC-3', antisense sequence: 3'-CGCUGAAUCUGACUGUGUUCA-5').

### Formulation of chitosan/siRNA nanoparticles

Chitosan was dissolved in 0.2 M sodium acetate buffer (adjusted to pH to 5.5 with sodium hydroxide) to a final concentration of 10 mg/mL (stock solution). 20 µl of siRNA (20 µm) (unless specifically specified) was diluted in RNAse free water (up to 100 or 200ul) and added to 800 µl or 900 ul of filtered chitosan (different concentration of chitosans, diluted from stock solution, was used to complex with siRNA at different N:P ratios.) whilst stirring and left for 1 hour.

### Size and surface charge determination of chitosan/siRNA nanoparticles

The size of the nanoparticles was determined by Photon Correlation Spectroscopy (PCS) and zeta potential by Laser Doppler Velocimetry (LDV) at 25°C using a Zetasizer Nano ZS. The size and zeta potential of nanoparticles formulated at different parameters are presented as the mean values of three measurements ± SD (standard deviation),

### Morphology observation of chitosan/siRNA nanoparticles by AFM

AFM imaging was performed with a commercial Digital Instruments Nanoscope IIIa MultiMode SPM (Veeco Instruments, Santa Barbara, CA) under ambient conditions. Samples were prepared by depositing a 1 µl drop of chitosan/siRNA nanoparticles solution on freshly cleaved mica surfaces and allowed to air dry. Standard V-shaped silicon tips (Ultra-sharp cantilevers, NSC11, MikroMasch Germany) were used with a resonance frequency of 45 kHz, a spring constant of 1.5N/m and a tip radius of < 10.0 nm. AFM images were obtained in tapping mode at 12 Hz scan rates. Images were flattened using the DI software algorithm (excluding the particles from the flattened area) and then analyzed automatically by using commercial Scanning Probe Image Processor (SPIP^{™}) software (Image Metrology ApS).

### Evaluation of the stability of chitosan/siRNA nanoparticles using polyacrylamide gel electrophoresis (PAGE)

Nanoparticle stability and siRNA integrity was investigated using a polydispacement assay. Samples were incubated +/- poly (L-aspartic acid) (PAA) (5mg/ml) at a 1:4 ratio (PAA: complex) at 37°C for 30 min. Samples were then analysed by electrophoresis using a 10% polyacrylamide gel (50 mM Tris-Borate, pH 7.9, 1 mM EDTA) at 150-230V for 2 h, stained with SYBR Gold nucleic acid stain and visualised using a UV illuminator.

### Gene silencing in EGFP expressed human cell line

H1299 green cells (half-life 2h) were plated on multiwell 24-plates (10⁵cells/well) in RPMI media (containing 10% fetal bovine serum (FBS), 5% penicillin/streptomycin, and 418 selection factor) 24 h prior to transfection. The media was removed and replaced with 250 µl serum-free media and the chitosan/siRNA nanoparticles or *Trans*IT-TKO transfection reagent added at 50 nM siRNA per well After 4 h, the media was replaced with 0.5 ml fresh media containing 10% FBS. The cells were left for 44 h and then removed using a standard trypsin protocol and resuspended in PBS containing 1% paraformaldehyde. The EGFP cell fluorescence was measured using a Becton Dickenson FACSCalibur flow cytometer.

### Evaluation of chitosan/siRNA nanoparticle cytotoxcity

Cellular cytotoxcity of chitosan/siRNA nanoparticles in H1299 green cells was determined using a tetrazolium-based viability assay. H1299 green cells in RPMI 1640 +GlutaMAX™I culture media supplemented with 10% FBS, 1% penicillin/streptomycin and G418, were seeded at a density of 10,000 cells/well in a 96-well plate in a total well volume of 100 µl, 24 h prior to assay. The media was removed and chitosan/siRNA nanoparticles added in triplicate and incubated for 4 h in serum-free medium (100 µl/well) after which the medium was replaced with medium containing 10% serum (100 µl/well). After 44 h, 20 µl of CellTitre 96 Aqueous proliferation assay solution (Promega Corporation, USA) was added to the plate and left for 3 h before absorbance measured at 490 nm using a 96-well plate reader(µQuant, Bio-Tek Instruments, Inc. USA).

### Results and discussion

### Effect of chitosan M_{w} and DD on size, zeta potential and morphology of chitosan/siRNA nanoparticles

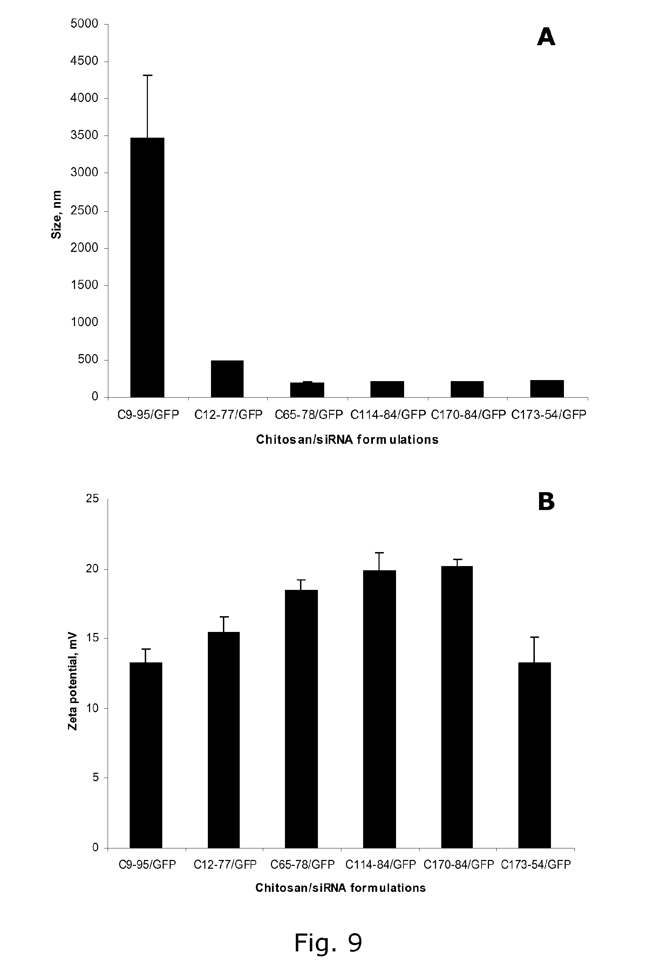

M_{w} and DD are important parameters for chitosan interaction with polyanionic species. The M_{w} correlates with the physical size of chitosan molecules, the high Mw forms are longer and more flexible molecules whereas the lower M_{w} forms are shorter and have stiffer molecular chains. The DD value signifies the percentage of deacetylated primary amine groups along the molecular chain, which subsequently determines the positive charge density when chitosan is dissolved in acidic conditions. Higher DD gives more positive charge and higher siRNA binding capacity. Chitosan samples with Mw ~ 10, 60, 100 and 170 kDa were prepared by depolymerisation of the commercially available chitosan product Chitopharm^{R}. As presented in Table 2, the 170 kDa polymer sample was also made available in a more acetylated form by controlled re-acetylation to reach a DD of 55%. Chitosan/siRNA nanoparticles were formulated at N:P ratio of 50 using different chitosan samples (Table 2) and the size and zeta potential of nanoparticles were studied (Fig 9).

**Table 2**

| **Chitosan samples with different Mw and DD** | | | | | | |
|---|---|---|---|---|---|---|
| Sample | C10-95* | C10-80 | C60-80 | C100-80 | C170-80 | C170-55 |
| Mw (kDa) | 10 | 10 | 60 | 100 | 170 | 170 |
| DD (%) | 95 | 80 | 80 | 80 | 80 | 55 |

| | | | | | | |
|---|---|---|---|---|---|---|
| *The numbers indicate Mw (molecular weight, kDa) and DD (the degree of deacetylation, %). | | | | | | |

Nanoparticles prepared using chitosan C9-95 (low M_{w} and high DD) measured ~ 3500 nm and decreased to ~ 500 nm with chitosan C12-77 (low M_{w} and medium DD). All other chitosan samples with medium and low DD (C60-78, C100-80, C170-80 and C170-55) resulted in particles with a size of approximately 200 nm (Fig 9A). The zeta potential of all formulations was in the range of 10 to 20 mV, suggesting a net positive surface charge due to excess chitosan (Fig 9B). The charge increased slightly with Mw, with the exception of C170-55 is lowered probably due to the reduced charge density (DD 55%)

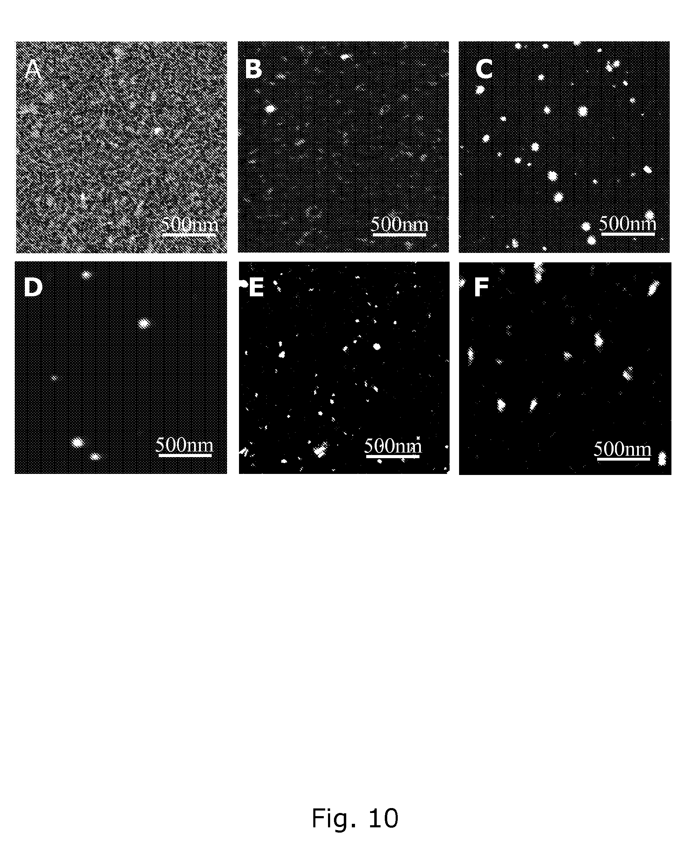

The formation of particles and the relative size was confirmed by AFM (Fig 10A-F). The large complexes formed by chitosan C10-95 could be due to shorter and stiffer chains that sterically hinder the interaction and incorporation of siRNA molecules resulting in bar- or circle-shape self-assembly and aggregation instead compact formations (Fig 10A). Chitosan C12-77 formed nanoparticles but with clear signs of aggregation (Fig 10B). Chitosan samples with higher Mw and medium DD (C60-80, C100-80 and C170-80) have sufficient chain length and charge density to complex and condense siRNA into nanoparticles (Fig 10C-E).

The siRNA molecule is composed of 21 bp with M_{w} of 13.36 kDa, which 5-10 times smaller than the chitosan molecules (M_{w} 60-170 kDa) used to form interpolyelectrolyte complexes. Thus, when mixed, the siRNA molecules are probably easily attracted by the electrostatic force of the protonated amine groups along the same or adjacent chitosan molecules resulting in chain connection and entanglement to form nanoparticles.

Chitosan sample C170-55 with lower DD and relative high Mw can also form nanoparticles (Fig 10F), which suggests that molecules with relatively low DD provide sufficient chain length and charge for complexing with siRNA. The hydrophobic interactions or hydrogen bonds between the glucosamine residues of chitosan and the specific structure of the organic bases of the nucleotide may also contribute to dense formation of nanoparticles.

### Effect of chitosan M_{w} and DD on the complex stability of chitosan/siRNA nanoparticles

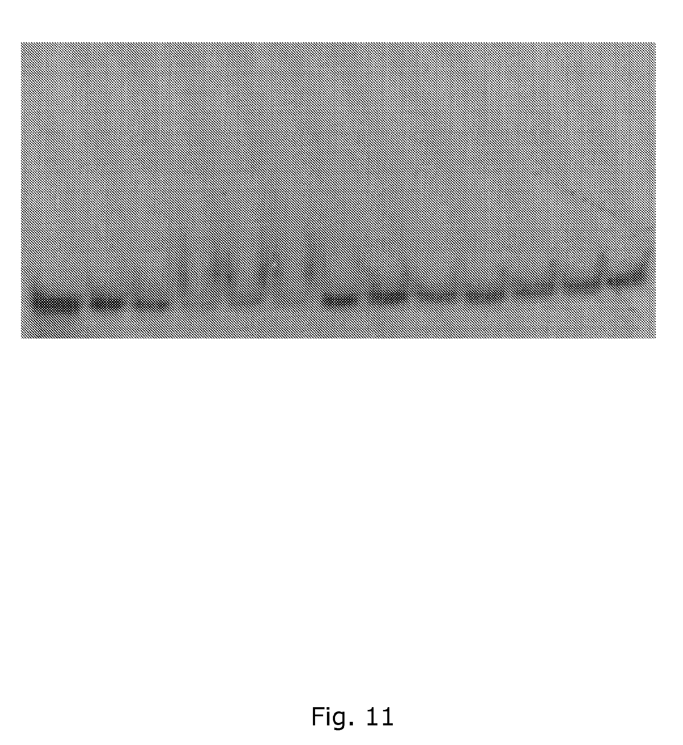

The influence of chitosan M_{w} and DD on particle stability was examined by observing the electrophoretic migration behaviour in the absence or presence of PAA (Fig 11). In general, high complex stability correlated with dense nano-size particle morphology shown with higher Mw and DD. When chitosan samples of C10-95 and C10-80 were used, the migration behaviour was almost the same as that of siRNA control (Fig 11, Lanes 1-3) displaying no retardation effect common with polyelectrolyte complexes. This suggests that chitosan with lower M_{w} of ~ 10 kDa (even with as higher DD as 95%) can not complex and compact siRNA into stable particles, which is in contrast to DNA plasmids over 24-mer (approximately 4.7 kDa) that form stable chitosan/DNA nanoparticles. An explanation could be that the longer DNA strands may be able to compensate for the shorter chitosan strands in the assembly process. No retardation effect on the migration of siRNA was observed when using C170-55 (Fig 11, Lane 7), which suggests the complexes formed with low deacetylated chitosan have less charge interaction and consequently unstable. Chitosan with greater charge density (C60-80, C100-80 and C170-80) retarded siRNA migration (Fig 11, Lanes 3-6) verifying the necessity of high charge for complex stability. In contrast, chitosan/DNA nanoparticles formed using chitosans of comparable M_{w} and DD show complete DNA retardation at lower N:P ratio of 2:1.

Nanoparticle stability is neccessary for extracellular siRNA protection; however, disassembly is essential in allowing RNA to be mediate gene silencing through interaction with intracellular components such as RISC. It has been noticed that highly stable chitosan/DNA complexes are beneficial to the protection of DNA in the cell endosomal-lysosomal pathway, but insufficient release of DNA from these particles to the nucleus reduce gene expression. This implies that a sufficient balance between protection and release is of great importance for appropriate biological functionality that may also apply to the delivery of siRNA.

Using a polyanion exchange method, siRNA was rapidly displaced from the complexes upon addition of polyanionic PAA. (Fig 11, Lanes 8-13). This suggests weak electrostatic interaction between chitosan and siRNA that may be disassociated under intracellular conditions allowing siRNA release and concomitant functionality. Importantly, the displaced siRNAs were structurally intact that confirmed maintained integrity of siRNA after nanoparticle formation.

### In vitro gene silencing and cytotoxcity of chitosan/siRNA nanoparticles

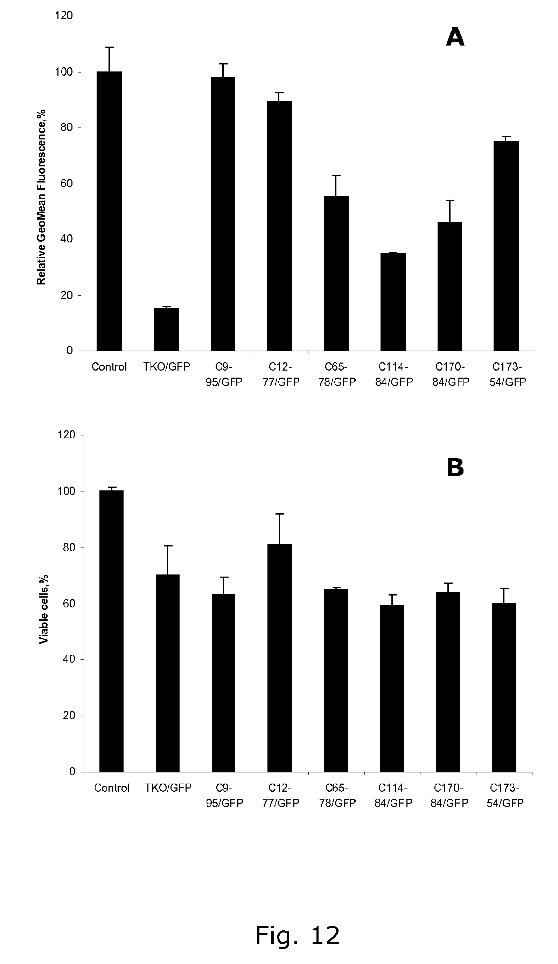

The stably expressing EGFP cell line (H1299 green cells) was used to investigate the gene silencing efficiency of the various chitosan/siRNA formulations. When compared to the non-transfected cells (negative control), chitosan/siRNA formulations (N:P 50) prepared with low M_{w} (C10-95, C10-80) or low DD (C170-55) showed almost no or low EGFP knockdown, whereas those prepared from higher M_{w} or DD showed greater gene silencing efficiencies of 45% (C60-80), 54% (C170-80) and 65% (C100-80). In comparison, the commercial *Trans*IT TKO (positive control) resulted in 85% EGFP knockdown. A cytotoxicity assay performed using the same chitosan formulation showed a 20-40% reduction in cell viability comparable with the commercial *Trans*IT TKO (Fig 12B).

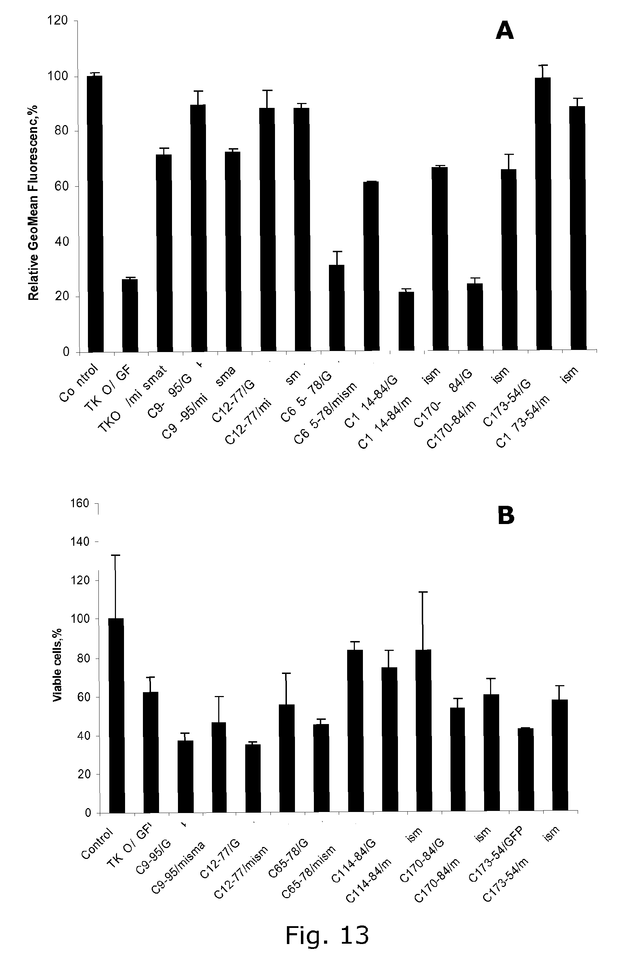

When the N:P ratio of chitosan/siRNA nanoparticles was increased to 150:1, gene silencing was increased to ~ 80% in formulations using high M_{w} chitosan (C100-80 and C170-80) formulations comparable with the commercial *Trans*IT TKO (Fig 13A). The specificity of knockdown was confirmed using mismatch siRNA formulations. In contrast, low levels of knockdown were again achieved with low M_{w} and DD chitosans. In general, a slight increase in cytotoxcity was found with formulations at N:P 150 compared to N:P 50 that may reflect the amount of free excess chitosan in the system. Levels, however, were no higher than those observed for the commercial *Trans*IT TKO (Fig 13B).

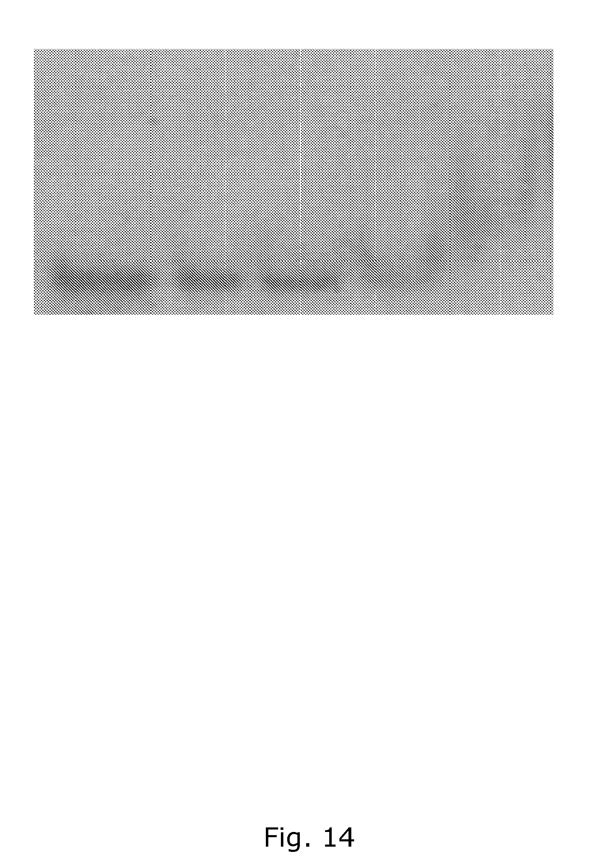

The biological knockdown results are fully consistent with the described physicochemical properties of chitosan/siRNA nanoparticles that suggest a correlation between the stability of the nanoparticles and gene silencing efficiency *in vitro*. Chitosan samples with high M_{w} in the range of 100-170 kDa and DD close to 80% are highly suitable for formulation of siRNA carrier systems. These formulations provide an efficient balance between appropriate protection and release, resulting in high *in vitro* gene silencing efficiencies equivalent to what can be achieved with existing commercial formulation systems. As discussed earlier, chitosan samples with high M_{w} and DD have longer molecular chains and higher charge density which enable efficient siRNA interaction and the formation of discrete nanoparticles. Nanoparticles formed with C100-80 and C170-80 have small size, higher surface charge and higher complex stability, which should enable them to become more easily endocytosed into cells and withstand endosomal-lysosomal conditions prior to cargo release resulting in higher gene silencing efficiency. Moreover, with the increase of N:P ratio to 150:1, the complex stability of nanoparticles increased indicated by the slower mobility of siRNA complexed with C170-80 (Fig 14). The increasing stability could be ascribed to increased levels of loosely bound chitosan at higher N:P. The excess chitosan loosely bound to the outer surface of nanoparticles may not only promote binding to anionic cell surfaces and subsequent endocytosis, but also provides protection against siRNA breakdown by lysosomal enzymes during the intracellular trafficking process. Exploitation of endosomolytic properties of chitosan allows siRNA entry into cytoplasm prior to interaction with RISC. Chitosan has been used to increase mucosal delivery; increased chitosan on nanoparticle surface can be exploited for mucosal RNAi applications.

### Conclusion

Interpolyelectrolyte complexes between chitosan and siRNA were used to form nanoparticles for siRNA delivery and gene silencing applications. Physicochemical properties such as size, zeta potential and complex stability of the nanoparticles were shown to be highly dependent on the structural parameters M_{w} and DD of the chitosan polymer. It was found that chitosan/siRNA nanoparticles formed using high M_{w}, (100-170 kDa) and DD (80%) forms of chitosan at N:P 150 were the most stable and exhibited the highest (~ 80%) *in vitro* gene knockdown. This work demonstrates the application of chitosan as a non-viral carrier for siRNA and the pivotal role of polymeric physical properties in the optimisation of gene silencing protocols.

### Example 3

This example shows morphological comparison of nanoparticles formed using direct additions of siRNA (concentrated method) or pre-diluted siRNA (diluted method) (Table 3).

**Table 3: Photon correlation spectroscopy of chitosan/siRNA nanoparticles prepared by concentrated or diluted method**

| Formulation | Size (nm)/Polydispersity Index |
|---|---|
| | |
| Chitosan (1mg/ml) 20µm* siRNA | 242.9/ 0.57 |
| Chitosan (1 mg/ml) 20µm siRNA | 193.0/ 0.37 |
| Chitosan (1mg/ml) 250 µm siRNA | 377.1/ 0.23 |
| | |
| Chitosan (250µg/ml) 20µm* siRNA | 821.2/ 0.83 |
| Chitosan (250µg/ml) 20µm siRNA | 206.6/ 0.25 |
| Chitosan (250µg/ml) 250 µm siRNA | 225.0/ 0.14 |

| | |
|---|---|
| *siRNA added in diluted volume; size average of 3 determinants | |

The experiment shows that using a RNA solution with a high concentration of RNA gives smaller particles with decreased polydispersity index compared to using a lower RNA concentration.

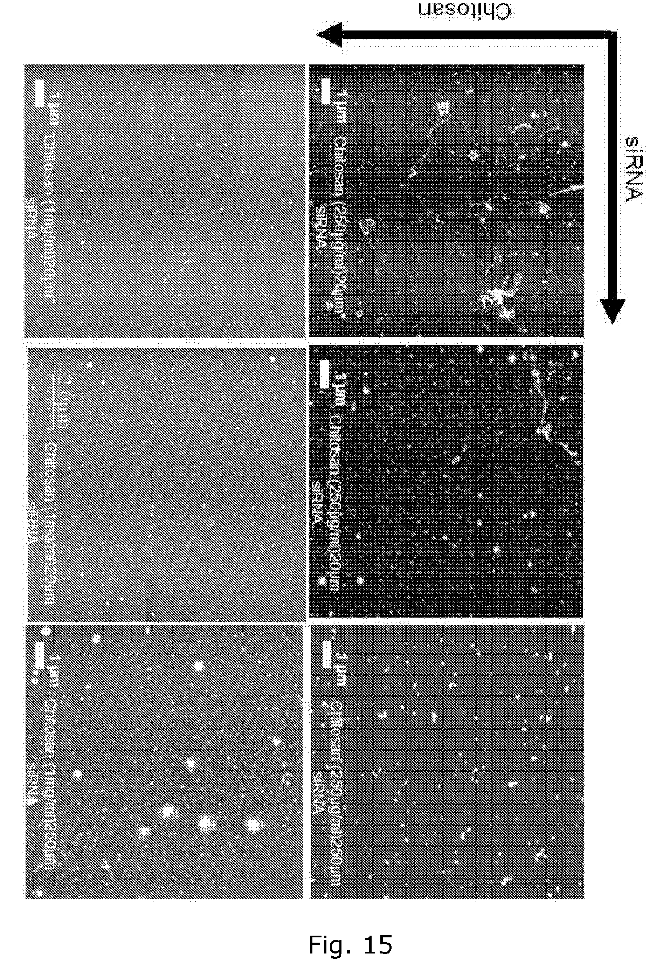

Atomic force microscopy effects of varying the amounts of chitosan and siRNA on nanoparticle morphology is shown if figure 15.

This shows more discrete formation of nanoparticles using the concentrated method.

### References

[1] Vårum KM, Ottøy MH, Smidsrød O. Water-solubility of partially N-acetylated chitosan as a function of pH: effect of chemical composition and depolymerization. Carbohydr Polym 1994;25: 65-70.
[2] Ottøy MH, Vårum KH, Christensen BE, Anthonsen MW, Smidsrød O. Preparative and analytical size-exclusion chromatography of chitosans. Carbohydr Polym 1996;31:253-61.
[3] Hirano S, Ohe Y, Ono H. Selective N-acetylation of chitosan. Carbohydr Res 1976;47:315-20.
[4] Muzzarelli RAA, Rocchetti R. Determination of the degree of acetylation of chitosans by first derivative ultraviolet spectrophotometry. Carbohydr Polym 1985;5:461-72.
[5] Khan TA, Peh KK, Ch'ng HS. Reporting degree of deacetylation values of chitosan: the influence of analytical methods. J Pharm Pharm Sci 2002;5:205-12.

SEQUENCE LISTING

<110> Aarhus Universitet
Besenbacher, Flemming
Howard, Kenneth Alan
Kjems, Jørgen
Liu, Xiudong

<120> Nanoparticles for nucleic acid delivery

<130> 40738EP03

<160> 10

<170> PatentIn version 3.4

<210> 1
<211> 21
<212> RNA
<213> Artificial

<220>
<223> RNA oligonucleotide

<400> 1
gacguaaacg gccacaaguu c 21

<210> 2
<211> 21
<212> RNA
<213> Artificial

<220>
<223> RNA oligonucleotide

<400> 2
acuuguggcc guuuacgucg c 21

<210>  3
<211>  20
<212>  RNA
<213>  Artificial

<220>
<223>  RNA oligonucleotide

<400>  3
gacguuagac ugacaaguuc                                                    20

<210>  4
<211>  23
<212>  RNA
<213>  Artificial

<220>
<223>  RNA oligonucleotide

<400>  4
acuugguguc cagucuaagu cgc                                                23

<210>  5
<211>  21
<212>  RNA
<213>  Artificial

<220>
<223>  RNA oligonucleotide

<400>  5
aagcagaguu caaaagcccu u                                                  21

<210>  6

<211>  21
<212>  RNA
<213>  Artificial

<220>
<223>  RNA oligonucleotide

<400>  6
aaggagaaua gcagaaugca u                                                21

<210>  7
<211>  21
<212>  RNA
<213>  Artificial

<220>
<223>  RNA oligonucleotide

<400>  7
gacguaaacg gccacaaguu c                                                21

<210>  8
<211>  21
<212>  RNA
<213>  Artificial

<220>
<223>  RNA oligonucleotide

<400>  8
acuuguggcc guuuacgucg c                                                21

<210>  9
<211>  21
<212>  RNA
<213>  Artificial

<220>
<223>  RNA oligonucleotide

<400>  9
gacuuagacu gacacaaguu c                                                21

<210>  10
<211>  21
<212>  RNA
<213>  Artificial

<220>
<223>  RNA oligonucleotide

<400>  10
cgcugaaucu gacuguguuc a                                                21

### Items

Item 1. A method of preparing a chitosan/RNA nanoparticle comprising the steps of:
   a. Providing a chitosan solution
   b. Providing an RNA solution
   c. Mixing the solution of step a with the solution of step b
   d. Incubating the solution of step c under conditions of complex formation such that chitosan/RNA nanoparticles form.
Item 2. A method according item 1, wherein the chitosan/RNA nanoparticle does not comprise an initial crosslinker.
Item 3. A method according to any of items 1 and 2, wherein the RNA solution comprises RNA at a concentration of at least 5 µM.
Item 4. A method according to any of the preceding items, wherein the chitosan solution comprises chitosan at a concentration of at least 50 µg/ml.
Item 5. A method according to any of the preceding items, wherein the nanoparticle is formed at a N:P ratio larger than 50.
Item 6. A method according to item 5, wherein the RNA solution comprises RNA at a concentration lower than 100 µM.
Item 7. A method according to any of items 5-6, wherein the formed nanoparticle comprises loosely bound chitosan.
Item 8. A method according any of items 5-7, where the nanoparticle with loosely bound chitosan is for mucosal delivery or for systemic delivery.
Item 9. A method according to any of items 1-4, wherein the N:P ratio is lower than 70.
Item 10. A method according to item 9, wherein the concentration of the RNA is higher than 100 µM.
Item 11. A method according to any of items 9-10, wherein the chitosan concentration is less than 250 µg/ml.
Item 12. A method according to any of items 9-11, wherein the size of the particle is between 10 and 500 nm.
Item 13. A method according to any of items 9-12, wherein the formed particles are discrete in form and have a polydispersity index lower than 0,4.
Item 14. A method according any of items 9-13, wherein the particle is for systemic delivery or for mucosal delivery.
Item 15. A method according to any of the preceding items, wherein the particle is for aerosol delivery.
Item 16. A method according to item 15, wherein the droplets of the aerosol are characterised in that they are smaller than 30 µm in diameter.
Item 17. A method according to any of the preceding items, wherein the RNA is a siRNA.
Item 18. A nanoparticle prepared by the method according to any of the preceding items.
Item 19. A method of treatment comprising
   e. Providing a nanoparticle prepared according to item 18
   f. Administrating said nanoparticle to a subject in need thereof.
Item 20. A method according to item 19, wherein the treatment comprise mucosal delivery of the nanoparticle.
Item 21. A method according to item 20, wherein the mucosal delivery is selected from the group of delivery to the respiratory tract, genitourinary delivery and oral delivery to oesophagus, stomach, small and large intestine.
Item 22. A nanoparticle according to item 18 for use as a medicament.
Item 23. Use of a nanoparticle according to item 18 for the preparation of a medicament for treatment of cancer, viral infection such as influenza, respiratory synthetic virus, and tuberculosis and inflammatory conditions such as arthritis, Crohn's disease, and hay fever.
Item 24. A method of delivering siRNA into a mammal or cell comprising
   g. Providing a nanoparticle according to item 18
   h. Subjecting said cell to the nanoparticle under conditions allowing entry of the siRNA.
Item 25. A method according to item 24, wherein the cell is a primary cell.
Item 26. A method according to item 24, wherein the cell is part of a mammal.
Item 27. A method according to item 24, wherein the mammal is non-human.
Item 28. A method of increasing the stability of a siRNA in a cell or in an organism comprising incorporation of the siRNA into a nanoparticle with chitosan by the method of any of items 1-17.
Item 29. A method of RNA interference in a cell or organism comprising the steps of
   i. Providing a nanoparticle according to item 18
   j. Subjecting said cell or organism to the nanoparticle under conditions allowing entry of the siRNA.
   k. Thereby mediating RNA interference of a gene corresponding to the siRNA of the nanoparticle.
Item 30. A method according to item 29, wherein the cell is a primary cell.

SEQUENCE LISTING

<110>  Aarhus Universitet
       Besenbacher, Flemming
       Howard, Kenneth Alan
       Kjems, Jørgen
       Liu, Xiudong

<120>  Nanoparticles for nucleic acid delivery

<130>  40738EP03

<160>  10

<170>  PatentIn version 3.4

<210>  1
<211>  21
<212>  RNA
<213>  Artificial

<220>
<223>  RNA oligonucleotide

<400>  1
gacguaaacg gccacaaguu c                                                21


<210>  2
<211>  21
<212>  RNA
<213>  Artificial

<220>
<223>  RNA oligonucleotide

<400>  2
acuuguggcc guuuacgucg c                                                21


<210>  3
<211>  20
<212>  RNA
<213>  Artificial

<220>
<223>  RNA oligonucleotide

<400>  3
gacguuagac ugacaaguuc                                                  20


<210>  4
<211>  23
<212>  RNA
<213>  Artificial

<220>
<223>  RNA oligonucleotide

<400>  4
acuugguguc cagucuaagu cgc                                              23


<210>  5
<211>  21
<212>  RNA
<213>  Artificial

<220>
<223>  RNA oligonucleotide

<400>  5
aagcagaguu caaaagcccu u                                                21


<210>  6
<211>  21
<212>  RNA
<213>  Artificial

<220>
<223>  RNA oligonucleotide

<400>  6
aaggagaaua gcagaaugca u                                                21


<210>  7
<211>  21
<212>  RNA
<213>  Artificial

<220>
<223>  RNA oligonucleotide

<400>  7
gacguaaacg gccacaaguu c                                                21


<210>  8
<211>  21
<212>  RNA
<213>  Artificial

<220>
<223>  RNA oligonucleotide

<400>  8
acuuguggcc guuuacgucg c                                                21


<210>  9
<211>  21
<212>  RNA
<213>  Artificial

<220>
<223>  RNA oligonucleotide

<400>  9
gacuuagacu gacacaaguu c                                                21


<210>  10
<211>  21
<212>  RNA
<213>  Artificial

<220>
<223>  RNA oligonucleotide

<400>  10
cgcugaaucu gacuguguuc a                                                21

## Claims

1. A chitosan/RNA nanoparticle, comprising RNA molecules of a length less than 100 nucleotides and chitosan.

2. The chitosan/RNA nanoparticle according to claim 1, wherein the RNA molecules are of a length less than 60 nucleotides.

3. The chitosan/RNA nanoparticle according to claims 1-2, wherein the RNA comprises RNA complexes of one or more RNA molecules, wherein the total number of nucleotides in the RNA complex is less than 100 nucleotides.

4. The chitosan/RNA nanoparticle according to claims 1-3, wherein the RNA molecules comprises chemical modifications selected from the group consisting of deoxynucleotides, LNA nucleotides, phosphorothioates.

5. The chitosan/RNA nanoparticle according to claims 1-4, there in the RNA is selected from the group consisting of a siRNA, an aptamer or a miRNA.

6. The chitosan/RNA nanoparticle according to claims 1-5, wherein the nanoparticle is for systemic delivery or for mucosal delivery.

7. The chitosan/RNA nanoparticle according to claims 1-6, wherein the nanoparticle is for aerosol delivery.

8. The chitosan/RNA nanoparticle according to claim 7, wherein the droplets of the aerosol are **characterised in that** they are smaller than 30 µm in diameter.

9. The chitosan/RNA nanoparticle according to claims 1-8 for use as a medicament.

10. The chitosan/RNA nanoparticle according to claims 1-9 for use in the treatment of cancer, viral infection such as influenza, and tuberculosis and inflammatory conditions such as arthritis, Crohn's disease, and hay fever.
